Europäisches Patentamt

European Patent Office

Office européen des brevets

(19)

(11) Numéro de publication: **0 255 408 B1**

## (12) FASCICULE DE BREVET EUROPEEN

(45) Date de publication de fascicule du brevet:
03.04.91

(51) Int. Cl.⁵: **A61F 2/08**

(21) Numéro de dépôt: **87400777.6**

(22) Date de dépôt: **07.04.87**

(54) Ligament artificiel synthétique imprégné et enrobé de résine élastique et son procédé d'enrobage.

(30) Priorité: **07.05.86 FR 8606655**

(43) Date de publication de la demande:
**03.02.88 Bulletin 88/05**

(45) Mention de la délivrance du brevet:
**03.04.91 Bulletin 91/14**

(84) Etats contractants désignés:
**AT BE CH DE ES FR GB GR IT LI LU NL SE**

(56) Documents cités:
**EP-A- 0 122 744**
**FR-A- 2 135 825**
**US-A- 3 805 300**
**US-A- 3 882 551**

(73) Titulaire: **Laboureau, Jacques-Philippe**
**24 rue de la Fontaine Billenois**
**F-21000 Dijon(FR)**

(72) Inventeur: **Laboureau, Jacques-Philippe**
**24 rue de la Fontaine Billenois**
**F-21000 Dijon(FR)**

(74) Mandataire: **Bruder, Michel**
**Cabinet Michel Bruder Conseil en Brevets**
**10, rue de la Pépinière**
**F-75008 Paris(FR)**

## Description

La présente invention concerne un ligament prothétique en textile synthétique et enrobé de résine élastique ainsi que le procédé de fabrication de ce ligament.

On connaît bien, chez les sportifs notamment. les accidents articulaires tels que la déchirure ou la rupture d'un ligament ; on sait aussi que dans la plupart des cas, certains de ces ligaments endommagés ne peuvent se reconstituer d'eux-mêmes.

La solution actuellement préconisée consiste en un remplacement du ligament fracturé par un ligament prothétique mis en place par intervention chirurgicale classique.

Dans ce type d'opération, on utilise le plus fréquemment des ligaments prothétiques réalisés en fibres synthétiques. Ces fibres sont disposées longitudinalement pour constituer l'âme résistante d'un tube de textile synthétique assurant à l'ensemble sa tenue mécanique.

De longueur et de diamètre convenables, en rapport avec le ligament endommagé que l'on prétend remplacer le tube de fibres textiles ainsi constitué présente indéniablement une résistance mécanique en traction satisfaisante.

Il convient toutefois d'observer qu'un empilage de fibres disposées longitudinalement, s'il satisfait, dans des proportions voulues, aux contraintes de traction que requiert au premier chef l'implant ligamentaire, ne peut en revanche supporter aucune surcharge en traction sans risque de déformation permanente. On comprend bien, en effet, qu'à la suite d'un effort de traction inhabituel, même momentané, sur un ligament prothétique du type précédemment décrit, les fibres disposées longitudinalement subissent un déplacement axial relatif, que rien ne peut raisonnablement empêcher, les fibres pouvant glisser les unes sur les autres. Le risque est alors grand de constater une déformation irréversible du ligament prothétique.

On peut donc résumer tout ceci en disant que les ligaments prothétiques actuellement disponibles, s'ils présentent de bonnes garanties de résistance aux efforts normaux d'une articulation comportant de telles prothèses, n'ont qu'une très médiocre capacité à supporter une contrainte violente même très fugitive et accidentelle.

Par ailleurs, il est fréquent d'observer qu'une implantation de ligament prothétique est mal tolérée par le patient et des réactions indésirables sont souvent à craindre car elles résultent du contact direct des fibres artificielles avec le milieu bilologique.

On connaît déjà quelques réalisations antérieures, telles que la solution décrite dans le brevet FR-A-2135825 où l'on propose de réaliser un tendon à partir d'un ruban de tricot indémaîllable en fibre polyester disposé à l'intérieur d'une gaine en élastomère indépendante du ruban. Cete réalisation apporte un caractère d'inextensibilité longitudinale et assure un meilleur coulissement du ruban dans sa gaine en élastomère, ce qui est, en principe, la fonction recherchée pour un tendon qui relie un muscle à un os.

De même, on connaît la solution accessoirement suggérée par le brevet US-A-3545008 qui préconise d'enrober les tendons et ligaments artificiels d'une résine élastomère biocompatible, de façon à isoler l'âme centrale du milieu biologique et éviter tout rejet; comme dans le brevet français précité, l'enrobage du tendon artificiel par un élastomère vient, en outre, faciliter le coulissement dudit tendon dans ses mouvements spécifiques.

On connaît encore le brevet EP-A-0051945 dans lequel est décrite une réalisation particulière d'un tendon ou ligament prothétique utilisant des élastomères tant pour la tenue mécanique des fibres entre elles, procurant des conformations diverses de la prothèse en fonction de ses applications, que pour assurer une biocompatibilité de ladite prothèse implantée. Le document US-A-3805300 décrit un tendon prothétique selon les characteristiques de préambule de la revendication 1.

Dans toutes ces réalisations antérieures utilisant en combinaison des textiles tricotés, enrobés par un élastomère, il n'est fait aucune mention du problème des déformations longitudinales des ligaments soumis à un effort momentané qui peut, d'ailleurs, être violent chez des sujets pratiquant un sport de compétition, par exemple.

On sait enfin qu'un ligament biologique peut subir une extension de 30% avant rupture correspondant à un effort voisin de 2000 newtons et qu'il procure assez généralement à l'individu une réserve d'extensibilité réversible, à l'occasion d'un effort soutenu correspondant à 1000 newtons, d'environ 18% sans aucun dommage causé audit ligament.

Suivant l'invention on a donc recherché une nouvelle combinaison de matériau tricoté et de résine élastomère procurant au ligament prothétique des propriétés mécaniques très proches de celles du ligament biologique que l'on prétend remplacer.

A cet effet ce ligament prothétique comportant une âme constituée de fibres synthétiques logitudinales adjacentes et torsadées enrobées d'un matériau visco-élastique biocompatible, à l'exception des parties extrêmes opposées de l'âme, les fibres étant maintenues torsadées par L'enrobage, est caractérisé par les caractéristiques de la partie caractérisante de la revendication 1.

Suivant une caractéristique complémentaire de l'invention l'enrobage de la partie centrale torsadée de l'âme obtenu pour imprégnation à sa température de fusion du matériau visco-élastique procure, d'une part, un effet d'élasticité longitudinale entièrement réversible pour des contraintes normalement acceptables par un ligament biologique et, d'autre part, un pouvoir d'élongation maximale à la rupture de l'ordre de 30% proche des caractéristiques du ligament biologique.

De préférence la zone centrale d'enrobage des fibres torsadées s'étend au moins sur toute la longueur du ligament correspondant à sa partie intra-articulaire. Ceci contribue, pour une très large part, à éviter les phénomènes d'intolérance lorsque le ligament est implanté sur le patient, en évitant le contact direct du matériau textile avec le milieu intra-articulaire, responsable, on le sait, de l'intolérance des prothèses. Naturellement, la résine utilisée pour cet enrobage doit être traitée, pour la rendre biocompatible, par les moyens et procédés habituels dans ce domaine.

Les extrémités du ligament prothétique destinées à demeurer dans l'os, pour obtenir la fixation dudit ligament, sont, par contre, dépourvues d'enrobage de résine, dont l'élasticité, les qualités de lubrification et l'état de surface sont incompatibles avec une réhabitation biologique dans le tunnel intra-osseux.

Les extrémités du ligament sont, de cette manière, fixées par tout moyen mécanique habituel, tel qu'agrafe chirurgicale, procurant audit ligament une première fixation artificielle. Ainsi enchâssées dans l'os les extrémités du ligament sont réhabitées par le même os qui, progressivement, emprisonne lesdites extrémités dont les surfaces, en contact avec l'os, présentent avantageusement toutes les anfractuosités du tricotage de l'âme textile du ligament, procurant ainsi la fixation définitive dudit ligament à l'os.

On décrira ci-après, à titre d'exemple non limitatif, une forme d'exécution de la présente invention, en référence au dessin annexé sur lequel :

La figure 1 est une vue en plan : de l'âme développée à plat destinée à faire partie du ligament prothétique suivant l'invention.

la figure 2 est une vue en perspective de l'âme après enroulement de celle-ci sur elle-même, autour d'un axe longitudinal.

La figure 3 est une vue en perspective de l'âme torsadée avant imprégnation et enrobage par la résine.

La figure 4 est une vue en perspective du ligament prothétique obtenu après imprégnation et durcissement de la résine élastomère.

La figure 5 représente un exemple d'implantation, entre deux os, d'un ligament prothétique suivant l'invention.

La figure 6 est une vue en coupe axiale d'un dispositif de moulage et d'imprégnation de la résine élastomère sur la partie intra-articulaire de l'âme torsadée, pour former le ligament prothétique suivant l'invention.

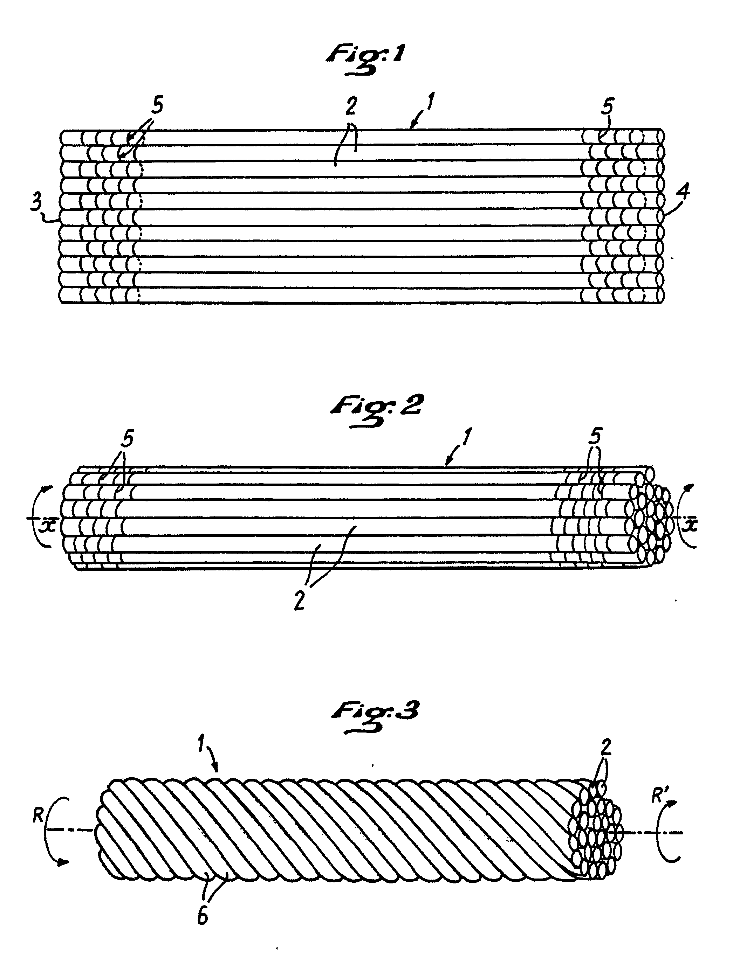

Si on se réfère à la figure 1, on voit que le ligament prothétique suivant l'invention est fabriqué à partir d'une âme 1 formée de fibres rectilignes adjacentes 2 disposées longitudinalement entre les deux extrémités 3 et 4 de l'âme 1. Ces fibres 2 sont de préférence réalisées en un matériau synthétique tel que le polyester dont on connaît les capacités propres d'extension de l'ordre de 10 à 15%. Les fibres 3 sont maintenues transversalement de toute façon appropriée, par exemple, par une colonne de mailles transversales 5 leur procurant un support du type à "mailles" jetées", comme il est représenté sur la figure 1. Les fibres 3 peuvent être maintenues entre elles sur toute la longueur de l'âme ou seulement dans les deux parties extrêmes opposées de celle-ci.

Le tricot ainsi constitué exclut toute déformation longitudinale de l'ensemble fibres rectilignes 2/mailles transversales 5 qui déterminerait alors une déformation irréversible et préjudiciable de l'âme 1 du ligament. De la sorte il ne subsiste que l'allongement propre aux fibres rectilignes 2 qui devient d'ailleurs irréversible au-delà d'un seuil de contrainte. En définitive les caractéristiques mécaniques des fibres rectilignes 2 ne sont en aucune manière affectées par leur mode de solidarisation.

L'âme 1 ainsi obtenue est enroulée sur elle-même, comme il est représenté sur la figure 2, autour de son axe longitudinal xx' en trois ou quatre passes concentriques et jointives, de manière à former un cylindre final, à section droite sensiblement en spirale, d'un diamètre externe sensiblement proche de celui du ligament biologique que l'on entend remplacer.

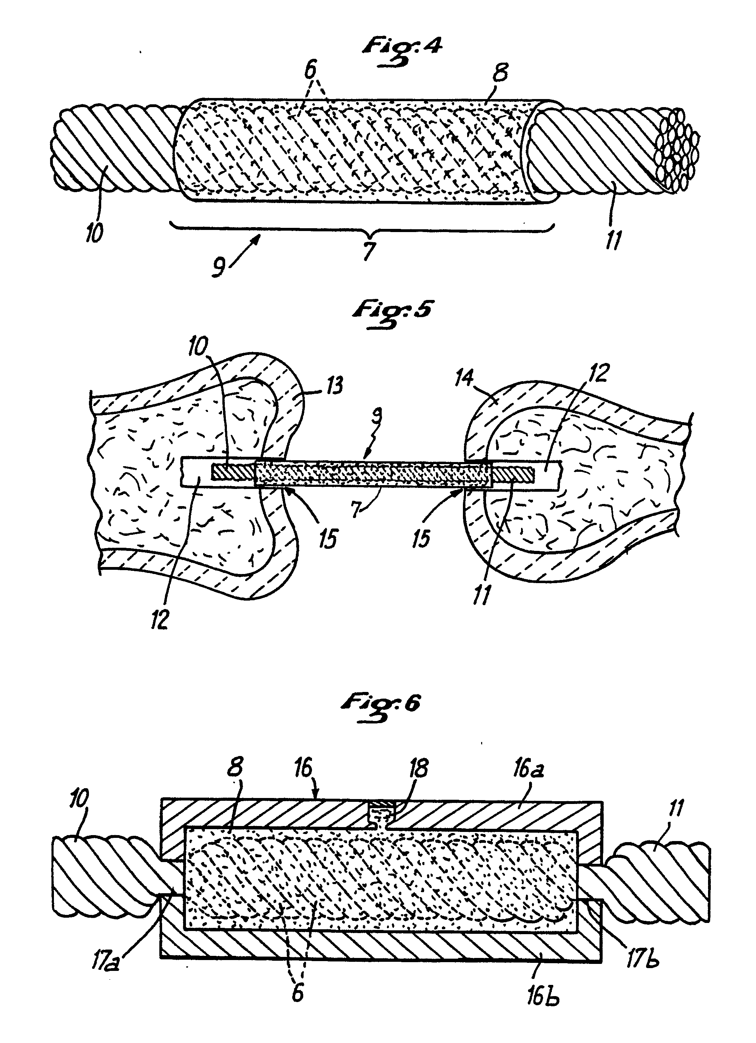

Ensuite, par des rotations inverses autour de l'axe longitudinal xx', dans le sens des flèches R et R' (figure 3), des extrémités 3 et 4 de l'âme 1 enroulée sur elle-même on forme des torsades 6, constituées par les fibres individuelles 2 enroulées en hélices adjacements. Ces torsades s'étendent sur toute la longueur de l'âme du ligament qui se trouve être raccourcie, du fait de sa torsion. Le nombre des spires des torsades 6 par unité de longueur ou le pas des hélices individuelles constituées par les fibres 2 détermine le pouvoir élastique du ligament prothétique final, ce pouvoir élastique dépendant de l'application recherchée.

L'âme 1 ainsi enroulée et torsadée est ensuite imprégnée et enrobée, dans sa seule partie centrale 7, destinée à constituer la partie intra-articulaire du ligament, au moyen d'une résine élastomère 8, (figure 4), par exemple du type polyuréthane, à une température sensiblement égale au point de fusion de la résine 8. On obtient ainsi un ligament prothétique 9 suivant l'invention dans lequel l'enrobage 8 de polyuréthane assure le maintien des torsades 6 sur toute la longueur de la zone centrale ou intra-articulaire 7, les deux parties extrêmes torsadées 10 et 11 de l'ame 1, qui ne sont pas imprégnées de résines, restant apparentes, de part et d'autre de la zone centrale 7 imprégnée de résine 8. Dans ces conditions on comprend qu'un effort de traction longitudinale entre les deux parties extrêmes 10 et 11 du ligament 9 sollicite, en priorité, les torsades 6 enrobées qui tendent à s'étirer, pour une remise en ligne. Réciproquement, dès que l'effort cesse, ces torsades 6 sont rappelées par l'effet de contrainte dû à l'imprégnation de résine élastomère 8. Par ailleurs au moment de l'imprégnation et de l'enrobage de la résine 8 à la température de fusion de cette résine, les fibres 2, alors hélicoïdales, de l'âme 1 du ligament subisse un retrait thermique, qui augmente, de la façon connue, leur pouvoir d'élongation. En effet les fibres 2, ainsi rétreintes thermiquement, présentent une première capacité d'allongement pour les ramener à leur position d'origine, à laquelle s'ajoute l'allongement naturel sous traction de la fibre non traitée. Ainsi observe t-on que, partant, d'une fibre non traitée thermiquement dont l'élongation naturelle, sous une traction donnée, est par exemple de 15%, on obtient une élongation complémentaire de 15% par rétraction thermique, ce qui procure à ladite fibre traitée un pouvoir d'élongation maximale proche de 30%.

En définitive le ligament prothétique 9 suivant l'invention présente, d'une part, un pouvoir d'élongation optimisé proche de l'élongation avant rupture du ligament biologique et, d'autre part, une élasticité qui résulte de l'addition de deux facteurs à savoir d'abord l'élasticité de la zone centrale torsadée et imprégnée 6 et ensuite l'élasticité propre des fibres 2 qu'on peut ainsi résumer :

- pour une traction longitudinale comprise entre 80 et 140 newtons appliquée entre les deux parties extrêmes 10 et 11 du ligament 9, seule la zone torsadée 7 dudit ligament absorbe l'effort en procurant un allongement moyen d'environ 12% du ligament 9; cet allongement est totalement réversible grâce à l'effet de rappel de la résine élastomère 8 dès lors que cesse l'effort de traction.

- pour une traction d'environ 1000 newtons qui correspond à un effort violent, quoique courant chez un sportif, pouvant être répété, le ligament prothétique 9 doit alors recourir en réserve à l'élasticité propre des fibres 2 de l'ordre de 6% pour des contraintes qui lui sont normalement appliquées, procurant au ligament prothétique 9 une élasticité globale de 18% suffisante pour "encaisser" un tel effort.

- pour une traction voisine de 2000 newtons le ligament prothétique 9 est à sa limite de rupture exactement à l'image du ligament biologique.

En définitive, le ligament prothétique torsadé 9 suivant l'invention, ayant des qualités de réponse élastique très proches du ligament biologique, procure l'avantage d'un double mécanisme de retour traitant les sollicitations fréquentes et quotidiennes des micro-traumatismes par le jeu de sa zone torsadée 7 en épargnant ainsi le travail des fibres 2 et en augmentant par conséquent leur durée de vie.

Suivant une caractéristique complémentaire du ligament prothétique 9, il est prévu d'enrober de résine la zone centrale du ligament sur une longueur supérieure à sa partie intra-articulaire de manière que les parties extrêmes de la zone centrale 7 enrobée de résine pénètrent quelque peu dans des tunnels 12 intra-osseux percés dans deux os en regard, comme on peut mieux le voir sur la figure 5. Cette disposition prévient tout cisaillement des fibres 2 constituant l'âme 1 du ligament par les bords 15 des tunnels 12.

L'enrobage du ligament torsadé 9 suivant l'invention, peut être avantageusement réalisé au moyen d'un moule 16 représenté sur ce moule 16 comprend deux demi-coquilles 16a,16b présentant chacune une cavité interne demi-cylindrique, d'un rayon un peu supérieur au rayon du ligament prothétique torsadé et de longueur légèrement supérieure à la partie intra-articulaire du même ligament à implanter. A chaque extrémité des deux demi-coquilles 16a,16b, un orifice 17a,17b, de diamètre légèrement inférieur à celui du ligament prothétique, perment de laisser à l'extérieur du moule les parties extrêmes 10,11 de l'âme qui ne doivent pas être enrobées de résine.

Enfin, grâce à un injecteur 18, on introduit dans le moule, dans au moins une demi-coquille 16a, la résine élastomère à base de silicone, par exemple du polyuréthane. Celle-ci, se présentant sous forme de pâte ou de feuille en fusion, s'étend dans le moule de façon uniforme et, après polymérisation, procure définitivement au ligament torsadé 9 ses qualités exceptionelles d'élasticité d'une part, et sa capacité à être toléré par l'organisme, d'autre part.

## Revendications

1. Ligament prothétique comportant une âme (1) constituée de fibres synthétiques (2) longitudinales adjacentes et torsadées, enrobées d'un matériau viscoélastique biocompatible, à l'exception des parties extrêmes opposées (10, 11) de l'âme (1), les fibres étant maintenues torsadées par L'enrobage, caractérisé en ce qu'avant la mise en torsade et l'enrobage du ligament (9) les fibres synthétiques (2), qui sont disposées longitudinalement de manière adjacente et rectiligne, sont maintenues solidaires les unes des autres, au moins à l'endroit des parties extrêmes (10, 11) de l'âme (1), par une colonne de mailles transversales (5) constituant un support du type tricot à "mailles jetées", n'introduisant aucune déformation longitudinale desdites fibres (2).

2. Ligament prothétique suivant la revendication précédente caractérisé en ce que l'enrobage de la partie centrale (7) torsadée de l'âme (1), obtenu par imprégnation à la température de fusion du matériau visco-élastique (8) procure, d'une part, un effet d'élasticité longitudinale entièrement réversible pour des contraintes normalement acceptables par un ligament biologique et, d'autre part, un pouvoir d'élongation maximale à la rupture de l'ordre de 30 %, proche des caractéristiques du ligament biologique.

3. Ligament prothétique suivant l'une quelconque des revendications précédentes caractérisé en ce que le matériau visco-élastique (8) est une résine du type polyuréthane.

4. Ligament prothétique suivant l'une quelconque des revendications précédentes, caractérisé en ce que la partie centrale (7) de l'âme qui est enrobée de résine (8) s'étend au moins sur toute la longueur du ligament (9) correspondant à sa partie intra-articulaire, les parties extrêmes intra-osseuses (10, 11) du ligament (9) qui ne sont pas enrobées de résine (8) assurant la réhabitation du ligament (9) par l'os.

5. Ligament prothétique suivant l'une quelconque des revendications précédentes caractérisé en ce que son âme (1) est constituée d'un tricot de fibres synthétiques (2, 5) enroulé longitudinalement sur lui-même en plusieurs passes concentriques et jointives.

6. Procédé de fabrication d'un ligament prothétique conforme à l'une ou l'autre des revendications 1 à 5 caractérisé en ce que l'âme (1) est formée à partir de fibres synthétiques (2), de préférence en résine polyester, rectilignes et adjacentes disposées longitudinalement et maintenues transversalement par une colonne de mailles transversales (5) formant un support du type tricot à "mailles Jetées", l'âme (1) ainsi obtenue est ensuite enroulée sur elle-même autour de son axe longitudinal en plusieurs passes concentriques et jointives pour obtenir un cylindre, de diamètre proche du ligament biologique, que l'on torsade par rotation inverse des extrémités (10, 11) suivant un nombre de torsades (6) dépendant du degré d'élasticité voulu, l'ensemble ainsi constitué est alors disposé en l'état dans un moule (16) constitué de deux demi-coquilles (16a , 16b ), présentant chacune une cavité interne demi-cylindrique, d'un rayon un peu supérieur au rayon du ligament prothétique torsadé (9) et de longueur légèrement supérieure à la partie intra-articulaire du même ligament à implanter, ce moule comportant, à chaque extrémité des deux demi-coquilles (16a , 16b ) un orifice (17a , 17b ) de diamètre légèrement inférieur à celui du ligament, lequel permet de laisser hors du moule (16) les parties extrêmes (10, 11) du ligament (9), et on injecte enfin dans au moins une demi-coquille (16a ) une résine élastomère (8), portée à sa température de fusion, laquelle s'étend uniformément autour de l'âme (1) du ligament et l'imprègne, l'âme (1) du ligament (9) étant maintenue torsadée à l'intérieur du moule (10).

7. Procédé suivant la revendication 6, caracterisé en ce qu'on effectue l'imprégnation à chaud de la resine (8) à sa température de fusion pour donner aux fibres (2) constituant la trame longitudinale de l'âme (1) du ligament (9) un retrait thermique venant augmenter de 10 à 15 % le pouvoir d'élongation des fibres (2).

**Claims**

1. Prosthetic ligament including a core (1) composed of longitudinal adjacent, twisted synthetic fibres (2), coated with a biocompatible visco-elastic material, except for the opposite extreme ends (10, 11) of the core (1), the fibres being maintained twisted by the coating, characterized in that before the ligament (9) is twisted and coated, the synthetic fibres (2), which are placed longitudinally in an adjacent and rectilinear manner, are kept joined together, at least at the extreme parts (10, 11) of the core (1) by a wale transversal stitches (5), composing a support such as warp knitting with weft insertion that does not cause any longitudinal deformation of the said fibres (2).

2. Prosthetic ligament in accordance with the previous claim, characterized in that the coating of the twisted central part (7) of the core (1), obtained by impregnation at melting temperature of the visco-elastic material (8) gives, on one hand, an entirely reversible longitudinal elastic effect for stresses that are normally acceptable by a biological ligament and, on the other hand, a tear strength maximum elongation approx. 30 %, close to the characteristics of a biological ligament.

3.  Prosthetic ligament according to any of the previous claims, characterized in that the visco-elastic material (8) is a polyurethane type resin.

4.  Prosthetic ligament according to any of the previous claims, characterized in that the central part (7) of the core, which is coated with resin (8), stretches at least over the length of the ligament (9) corresponding with its intra-articular part, the extreme intra-bone parts (10, 11) of the ligament (9) which are not coated with resin (8) assuring the rehabitation of the ligament (9) by the bone.

5.  Prosthetic ligament according to any of the previous claims characterized in that its core (1) is composed of a synthetic fibre knit (2, 5) rolled longitudinally over itself in several concentric, joining turns.

6.  Manufacturing process for a prosthetic ligament in accordance with any of the claims 1 to 5, characterized in that the core (1) is formed of synthetic fibres (2) preferably of polyester resin, straight and adjacent, laid longitudinally and held transversally by a wale of transversal stitches (5) forming a support of warp knitting with weft insertion, the core (1) thus obtained then being rolled round itself, around its longitudinal axis, with several concentric joining turns to obtain a cylinder with a diameter close to that of a biological ligament which is twisted by the inverse rotation of the extremities (10, 11) according to a number of twists (6) depending on the degree of elasticity required, the unit thus obtained then being placed as it is in a mold (16) composed of two half-shells (16a , 16b) each offering a semi-cylindrical internal cavity with a radius slightly higher than the twisted prothetic ligament (9) and slightly longer at the intra-articular part of the same ligament to be implanted, this mold including at each end of the two half-shells (16a, 16b ) a hole (17a, 17b) with a diameter slightly smaller than that of the ligament, which allows the extreme parts (10, 11) of the ligament (9) to be left outside the mold (16) and, finally, an elastomere resin (8) is injected into at least half of a shell (16a ), the said resin being brought to its melting point, which will uniformally spread around the core (1) of the ligament and impregnate it, the core (1) of the ligament (9) being maintained twisted inside the mold (16).

7.  Process according to claim 6, characterized in that the hot impregnation of the resin (8) at its melting temperature is carried out to give the fibres (2) composing the longitudinal weave of the core (1) of the ligament (9), a thermal contraction increasing by 10 to 15 % the elongation capacity of the fibres (2).

## Ansprüche

1.  Bänderprothese mit einer Seele (1) aus longitudinalen, benachbarten und verdrillten Kunststoffasern (2), welche mit Ausnahme der entgegengesetzten Enden (10, 11) der Seele (1) mit einem viskoelastischen, gewebeverträglichen Material umhüllt sind und durch die Umhüllung verdrillt gehalten werden, **dadurch gekennzeichnet,** daß vor der Verdrillung und Umhüllung des Bandes (9) die Kunststoffasern (2), die longitudinal nebeneinander und geradlinig angeordnet sind, miteinander zumindest bei den Enden (10, 11) der Seele (1) durch eine Reihe von Quermaschen (5) zusammengehalten werden, welche einen Träger nach Art eines "Umschlagmaschen"-Gewirks bilden und keinerlei longitudinale Verformung der Fasern (2) verursachen.

2.  Bänderprothese gemäß dem vorangehenden Anspruch, **dadurch gekennzeichnet,** daß die Umhüllung des mittleren verdrillten Teiles (7) der Seele (1), die durch Tränken bei der Schmelztemperatur des viskoelastischen Materials (8) erhalten wurde, einerseits eine völlig reversible longitudinale Elastizität für Beanspruchungen, wie sie normalerweise für ein natürliches Band tragbar sind, und andererseits die Fähigkeit einer maximalen Reißlänge in der Größenordnung von 30%, ähnlich den Eigenschaften eines natürlichen Bandes ergibt.

3.  Bänderprothese nach einem der vorhergehenden Ansprüche, **dadurch gekennzeichnet,** daß das viskoelastische Material (8) ein polyurethankunststoff ist.

4.  Bänderprothese nach einem der vorhergehenden Ansprüche, **dadurch gekennzeichnet,** daß der mittlere Teil (7) der Seele, der mit dem Kunststoff (8) umhüllt ist, sich über mindestens die gesamte Länge des Bandes (9) erstreckt, die dessen intraartikulärem Teil entspricht, und daß die intraossalen Endteile (10, 11) des Bandes, die nicht mit Kunststoff (8) umhüllt sind, die Aufnahme des Bandes (9) durch den Knochen gewährleisten.

5. Bänderprothese nach einem der vorhergehenden Ansprüche, **dadurch gekennzeichnet,** daß ihre Seele (1) aus einem Gewirk aus Kunststoffasern (2, 5) besteht, das longitudinal zu mehreren konzentrischen und dichten Lagen zusammengerollt ist.

6. Verfahren zum Herstellen einer Bänderprothese gemäß einem der Ansprüche 1 bis 5, **dadurch gekennzeichnet,** daß die Seele (1) ausgehend von vorzugsweise aus Polyesterharz bestehenden Kunststoffasern (2) gebildet wird, die geradlinig und nebeneinander in Längsrichtung angeordnet und in Querrichtung durch eine Reihe von Quermaschen (5) gehaltert sind, die einen Träger nach Art eines Umschlagmaschengewirks bilden, daß die so erhaltene Seele (1) um ihre Längsachse zu mehreren konzentrischen und dichten Lagen aufgewickelt wird, um einen Zylinder mit einem Durchmesser ähnlich dem eines natürlichen Bandes zu erhalten, den man durch entgegengesetzte Drehung der Enden (10, 11) mit einer Anzahl von Drehungen (6) in Abhängigkeit von dem gewünschten Grad von Elastizität verdrillt, daß die so gebildete Anordnung in diesem Zustand in eine Form (16) eingebracht wird, die aus zwei Halbschalen (16a, 16b) mit jeweils einem halbzylinderförmigen Hohlraum besteht, dessen Radius etwas größer als der der verdrillten Bänderprothese (9) und dessen Länge etwas größer als der intraartikuläre Teil des zu implantierenden Bandes ist, wobei die Form an jedem Ende der Halbschalen (16a, 16b) jeweils eine Öffnung (17a, 17b) hat, deren Durchmesser etwas kleiner ist als der des Bandes und die es gestatten, die Enden (10, 11) des Bandes (9) außerhalb der Form (16) zu belassen, und daß man schließlich in mindestens eine Halbschale (16a) einen elastomeren Kunststoff (8) einspritzt, der auf seine Schmelztemperatur gebracht ist und der sich gleichmäßig um die Seele (1) des Bandes verteilt und diese tränkt, wobei die Seele (1) des Bandes (9) im Inneren der Form (16) verdrillt gehalten wird.

7. Verfahren nach Anspruch 6, **dadurch gekennzeichnet,** daß man die Heißtränkung mit dem Kunststoff (8) bei dessen Schmelztemperatur bewirkt, um den Fasern (12), welche den longitudinalen Schuß der Seele (1) des Bandes (9) bilden, eine Wärmeschrumpfung zu erteilen, die die Dehnungsfähigkeit der Fasern (2) um 10 bis 15% erhöht.

*Fig:1*

*Fig:2*

*Fig:3*

*Fig:4*

*Fig:5*

*Fig:6*